(19) Europäisches Patentamt
European Patent Office
Office européen des brevets

(11) Veröffentlichungsnummer: **0 005 223**
**B1**

# (12) EUROPÄISCHE PATENTSCHRIFT

(45) Veröffentlichungstag der Patentschrift: **21.09.83**

(51) Int. Cl.³: **C 07 C 91/26**

(21) Anmeldenummer: **79101209.9**

(22) Anmeldetag: **21.04.79**

(54) **Verfahren zur Herstellung von 3-Halogen-2-hydroxialkyl-tri-alkylammoniumchlorid.**

(30) Priorität: **05.05.78 NL 7804880**

(43) Veröffentlichungstag der Anmeldung:
**14.11.79 Patentblatt 79/23**

(45) Bekanntmachung des Hinweises auf die Patenterteilung:
**21.09.83 Patentblatt 83/38**

(84) Benannte Vertragsstaaten:
**BE DE FR GB IT LU SE**

(56) Entgegenhaltungen:
**CHEMISCHE BERICHTE, Band 106, Nr. 5, 1973, Weinheim, DE, J. BUDDRUS et al.: «Dehydrohalogenierung von Halogenalkanen mit Epoxiden in Gegenwart von Halogenid-Ionen», Seiten 1648–1660**

(73) Patentinhaber: **Chemische Fabriek Zaltbommel B.V., Postbus 52 Koxkampseweg 14-20, Zaltbommel (NL)**

(72) Erfinder: **Houben, Marie Joseph Francicus, Brabantlaan 67, Vught (NL)**
Erfinder: **Van der Maas, Hendrikus Johannes Hubertus, de Boogerd 25, Zuilichem (NL)**

(74) Vertreter: **Siecke, Wolf-Friedrich, Dr., c/o DYNAMIT NOBEL AKTIENGESELLSCHAFT HA Patente/Patentabteilung Postfach 1209 Kölner Strasse, D-5210 Troisdorf/Bez. Köln (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 005 223 B1

# Verfahren zur Herstellung von 3-Halogen-2-hydroxialkyl-trialkylammoniumchlorid

Gegenstand der vorliegenden Erfindung ist ein Verfahren zur Herstellung von 3-Halogen-2-hydroxialkyl-trialkyl-ammoniumchloriden, bei dem ein 1-Halogen-2,3-epoxialkan mit Trialkylaminen umgesetzt und eine Spaltung des Epoxirings durchgeführt wird.

Die Umsetzung von 1-Halogen-2,3-epoxipropan mit Trialkylaminen ist z. B. aus der US-PS 2876217 bekannt. Dabei entstehen die entsprechenden epoxigruppenhaltigen Trialkylammoniumhalogenide in Form einer wässrigen Lösung, die direkt zur Reaktion mit Stärke eingesetzt wird. Das Umsetzungsprodukt mit Stärke verhindert eine vorzeitige Gelatinierung letzterer und ermöglicht die Herstellung von Stärke in ausgeflockter Form.

Nachteilig bei der in der US-PS 2876217 beschriebenen Verfahrensweise ist das Arbeiten in wässrigen Milieu, das zu einer Reihe von Nebenreaktionen führt, die die Reaktion mit der Stärke ungünstig beeinflussen. Es wurde deshalb auch bereits vorgeschlagen, die Herstellung des epoxidgruppenhaltigen Tryalkylammoniumhalogenids in einem wasserfreien organischen Lösungsmittel durchzuführen, um zu einem wasserfreien Produkt zu gelangen , das man bei der Herstellung als Niederschlag abfiltrieren kann und das dann in flockiger Form vorliegt (vgl. DE-PS 1518330 = NL-PS 151081 und DE-OS 2056002 = NOA 7115436). Besonders nach dem Verfahren des zuletzt genannten Schutzrechtes erhält man die gewünschten Produkte in hohen Ausbeuten und in leicht abtrennbarer Form. Nachteilig wirkt sich bei diesem Verfahren die Tatsache aus, dass im wasserfreien Medium gearbeitet werden muss, und Wassergehalte über 2% zu Zersetzungen führen. Man muss desshalb wegen der starken Hygroskopie der Verbindungen Massnahmen durchführen, um den Zutritt von Luftfeuchtigkeit zu verhindern, da sonst das fertige Produkt zusammenklumpt.

Die Herstellung der 3-Halogen-2-hydroxi-trialkylammoniumhalogenide erfolgt deshalb immer noch aus wässriger Lösung entsprechend der in der US-PS 2876217 beschriebenen Verfahrensweise, indem das Reaktionsprodukt aus Trialkylamin und 1-Halogen-2,3-epoxipropan mit Salzsäure umgesetzt wird. Dabei entsteht als Nebenprodukt in Mengen bis zu 20% das Halogenid des Bistrialkylammonium-2-hydroxipropans. Dieses Nebenprodukt fällt mit aus und reagiert nicht mit Stärke, während das zu etwa 80% entstehende 3-Halogen-2-hydroxi-trialkylammoniumhalogenid ebenfalls zur Veresterung von Stärke und damit zur Stabilisierung letzterer eingesetzt wird.

Weiterhin entsteht bei der Verfahrensweise der US-PS 2876217 durch Reaktion des Epichlorhydrins mit Salzsäure 1,3-Dichlorpropanol-2, welches mit Stärke unter Vernetzung reagiert. Der Gehalt an diesem Propanol muss in dem gewünschten Endprodukt deshalb unter 50 ppm liegen.

Es bestand deshalb die Aufgabe, 3-Halogen-2-hydroxialkyl-trialkylammoniumhalogenide so herzustellen, dass die Bildung der oben genannten Bis-Verbindung und des 1,3-Dichlor-propanol-2 unterbunden wird und das gewünschte Endprodukt in einer Reinheit von mindestens 97% als kristallines Produkt direkt anfällt.

In Erfüllung dieser Aufgabe wurde nun ein Verfahren zur Herstellung von 3-Halogen-2-hydroxialkyltrialkylammoniumhalogeniden gefunden, bei dem eine Oxiran-Gruppe mit einem Trialkylamin zur Umsetzung gebracht wird, das dadurch gekennzeichnet ist, dass man als Verbindung mit einer Oxiran-Gruppe Chloralkyläthylenoxide einsetzt und vor oder während der Reaktion Tetrachloräthan hinzufügt.

Bei Durchführung der erfindungsgemässen Verfahrensweise erhält man Ausbeuten an dem gewünschten Endprodukt von über 90%. Das erhaltene Produkt hat eine Reinheit von über 97% und braucht vor seinem weiteren Einsatz keiner weiteren Reinigungsoperation unterworfen zu werden. Es enthält weder die oben genannte Bis-Verbindung noch 1,3-Dichlorpropanol-2.

Die erfindungsgemässe Reaktion läuft bereits bei Raumtemperatur ab. Ein Erhitzen auf Temperaturen bis etwa 80°C ist durchaus möglich, aber nicht verfahrenswesentlich.

Die Menge des eingesetzten Chloralkyläthylenoxids sollte mindestens äquivalent der Menge des Trialkylamins sein; d.h. pro Mol Trialkylamin soll mindestens ein Mol Chloralkyläthylenoxid eingesetzt werden. Es ist jedoch vorteilhaft, das Chloralkyläthylenoxid in einem geringen Überschuss einzusetzen, der in der gleichen Grössenordnung wie derjenige des Tetrachloräthans liegt.

Auch das Tetrachloräthan soll im Überschuss zu dem Trialkylamin eingesetzt werden. Pro Mol Trialkylamin soll mindestens ein Mol Tetrachloräthan eingesetzt werden. Es empfiehlt sich jedoch ein Überschuss von etwa 3 bis 10 Mol %.

Bei dem einzusetzenden Tetrachloräthan handelt es sich überwiegend um das symmetrische Isomere, 1,1,2,2-Tetrachloräthan. Dieses dient nicht nur als Lösungsmittel, sondern nimmt selbst an der Reaktion teil. Es wird vermutet, dass folgende Reaktionen ablaufen:

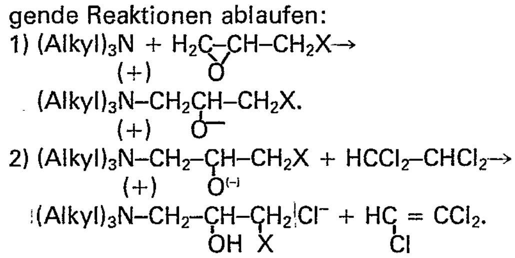

Für einen Reaktionsablauf entsprechend diesen Gleichungen spricht der Nachweis von Trichloräthylen in der erhaltenen Mutterlauge. Die Zwischenprodukte konnten nicht nachgewiesen werden. Sie brauchen auch nicht isoliert zu werden, so dass sämtliche Reaktionspartner gleichzeitig in dem Reaktionsgefäss anwesend sein können. Eine

Reaktion zwischen Tetrachloräthan und dem Epoxialkan findet nicht statt.

Im allgemeinen geht man bei der Durchführung des erfindungsgemässen Verfahrens so vor, dass man Tetrachloräthan zusammen mit dem Epoxialkan vorlegt und das Trialkylamin hinzufügt. Es ist jedoch auch möglich, Trialkylamin und Tetrachloräthan zusammen vorzulegen und das Epoxialkan dem Gemisch hinzuzufügen. In allen Fällen entsteht nach einiger Zeit ein Niederschlag aus dem gewünschten 3-Halogen-2-hydroxialkyl-trialkylammoniumhalogenid, der dann nach an sich bekannten Methoden gereinigt, abgetrennt und getrocknet werden kann.

Im allgemeinen genügt es, als Lösungsmittel das Tetrachloräthan bzw. das daraus entstandene Trichloräthylen zu verwenden. Es ist jedoch prinzipiell auch möglich, die Reaktion in Gegenwart eines zusätzlichen Lösungsmittels durchzuführen. Als Lösungsmittel eignen sich z.B. Mischungen von Tetrachloräthan mit Chlorkohlenwasserstoffen, gesättigte Kohlenwasserstoffe mit mehr als 5 C-Atomen und Alkylketone mit 3 bis 6 C-Atomen.

Als Chloralkyläthylenoxid wird bevorzugt 1-Chlor-2,3-epoxipropan (Epichlorhydrin) eingesetzt. Man erhält dann das 3-Chlor-2-hydroxipropyl-trialkylammoniumchlorid. Die Reaktion lässt sich jedoch auch mit den entsprechenden Brom- und Jodepoxipropanen durchführen. Prinzipiell lassen sich nach der erfindungsgemässen Reaktion auch 3-Halogen-2-hydroxialkyl-trialkylammoniumhalogenide herstellen, bei denen als Ausgangsprodukt ein Chloralkyläthylenoxid eingesetzt wird, dessen Chloratom in α-Stellung zur Äthylenoxidgruppe steht und dessen Alkylgruppe bis zu 4 C-Atomen besitzt.

Das nach dem neuen Verfahren bevorzugt hergestellte 3-Chlor-2-hydroxipropyl-trimethylammoniumchlorid ist in Aceton und Hexan unlöslich. Beim Auflösen in Natronlauge bildet sich die 2,3-Epoxiverbindung, die wiederum zur Verätherung von Stärke und der Bildung von kationischen Polyelektrolyten dient.

Beispiel 1

Herstellung von 3-Chlor-2-hydroxipropyl-N,N,N-Trimethyl-ammoniumchlorid. In einem Flanschkolben mit einem 3-Liter Inhalt, mit Rührer, sowie mit Thermometer und einem Gaszufuhrrohr versehen, werden 1600 $cm^3$ 1,1,2,2-Tetrachloräthan eingeleitet. Davon wurde unter Rühren 508,7 g (= 431 $cm^3$) Epichlorhydrin zugesetzt. Via GLC wurde die Zusammensetzung in Gew.-% bestimmt. Man liess das Gemisch über Nacht bei Raumtemperatur stehen. Via GLC wurde nachgewiesen, dass die Zusammensetzung noch die Gleiche war, c.q. es gab keine Reaktion des Epichlorhydrins mit Tetrachloräthan unter diesen Umständen. Dieser Mischung wurde unter Rühren Trimethylamin in Gasform zugesetzt. Es wurden insgesamt 273,2 g zugesetzt unter Kühlung bei 18–23°C. Nach etwa einer Stunde wird die Reaktionsmischung trübe und bilden sich Kristalle. Nach der Zusetzung wurde noch 24 Stunden lang nachgerührt. Der gebildete Kristallbrei wurde abfiltriert und mit Tetrachloräthan nachgewaschen. Anschliessend mit Heptan gewaschen und getrocknet. Insgesamt wurden 283,2 g Trockenmaterial erhalten. Via NMR-Analyse wurde nachgewiesen, dass die gebildete Verbindung das 3-Chlor-2-hydroxipropyl-N,N,N-Trimethylammoniumchlorid grosser Reinheit war. Analyse des Stoffes gab die folgenden Ergebnisse.

| Gefunden: | | Literatur: |
|---|---|---|
| Schmelzpunkt | 187–188°C | 187–189°C |
| Stickstoffgehalt | 6,96–7,27% | 7,44% |
| Chlorid | 18,86–18,89% | 18,85% |
| Total an Chlor | 37,0–37,4% | 37,70% |
| Chlorhydringehalt | 98,8% | |

In der abfiltrierten Mutterlauge bildeten sich noch einige Kristalle. Via GLC konnte in der Mutterlauge Trichloräthylen nachgewiesen werden.

Beispiel 2

In einem 3-Liter Kolben wurden 2498 g der Mutterlauge aus dem Beispiel 1 (Gehalt an 1-Chlor-2,3-epoxipropan = 2,6 Gew.-% 64,5 g) mit 427,8 g 1-Chlor-2,3-epoxipropan und 100 ml (= 160 g) 1,1,2,2-Tetrachloräthan versetzt. In diese Lösung wurde bei Raumtemperatur insgesamt 264,6 g Trimethylamin mit einer solchen Geschwindigkeit eingeleitet, dass kein gasförmiges Trimethylamin entweichen konnte.

Nach etwa einer Stunde bildeten sich die ersten Kristalle. Das Reaktionsgemisch wurde etwa 15 Stunden lang (über Nacht) gerührt. Anschliessend wurden die ausgefallenen Kristalle abfiltriert und mit Tetrachloräthan gewaschen. Die darauffolgende Trocknung erfolgte im Wasserbad von 100°C bei einem Vakuum von etwa 2 mbar.

Es wurden 748 g 3-Chlor-2-hydroxipropyl-trimethylammoniumchlorid vom Schmelzpunkt 187,6 bis 188°C erhalten. Der Chlorhydringehalt beträgt 99,3%.

**Patentansprüche**

1. Verfahren zur Herstellung von 3-Halogen-2-hydroxialkyltrialkylammoniumchloriden, bei dem eine Oxiran-Gruppe mit einem Trialkylamin zur Umsetzung gebracht wird, dadurch gekennzeichnet, dass man als Verbindung mit einer Oxiran-Gruppe Chloralkyläthylenoxide einsetzt und vor oder während der Reaktion Tetrachloräthan hinzufügt.

2. Verfahren gemäss Anspruch 1, dadurch gekennzeichnet, dass man das Trialkylamin den vorgelegten übrigen Reaktionspartnern hinzufügt.

3. Verfahren gemäss Ansprüchen 1 oder 2, dadurch gekennzeichnet, dass die Reaktion mit überschüssigem Chloralkyläthylenoxid, bezogen auf eingesetztes Trialkylamin, durchgeführt wird.

4. Verfahren gemäss einem der Ansprüche 1 bis 3, dadurch gekennzeichnet, dass die Reaktion mit überschüssigem Tetrachloräthan, bezogen auf eingesetztes Trialkylamin durchgeführt wird.

5. Verfahren gemäss einem der Ansprüche 1 bis 4, dadurch gekennzeichnet, dass man die Umsetzung in einem organischen Lösungsmittel durchführt.

## Claims

1. Process for the preparation of 3-halogen-2-hydroxyalkyltrialkylammonium chlorides in which an oxirane group is brought to reaction with a trialkylamine, characterized in that chloroalkylethylene oxide is employed as compound with an oxirane group and before or during the reaction tetrachloroethane is added.

2. Process according to claim 1, characterized in that the trialkylamine is added to the remaining reaction partners which have been previously emplaced.

3. Process according to claims 1 or 2, characterized in that the reaction is carried out with excess chloroalkylethylene oxide, related to the trialkylamine employed.

4. Process according to one of claims 1 to 3, characterized in that the reaction is carried out with excess tetrachloroethane, related to trialkylamine employed.

5. Process according to one of claims 1 to 4, characterized in that the reaction is carried out in an organic solvent.

## Revendications

1. Procédé de préparation de chlorures de 3-halogéno-2-hydroxy-alkyl-trialkyl-ammonium, procédé dans lequel on fait réagir un groupe oxiranne avec une trialkylamine, caractérisé en ce que, comme composés comportant un groupe oxiranne, on utilise des oxydes de chloralkyléthylène, et l'on ajoute du tétrachloréthane avant ou pendant la réaction.

2. Procédé suivant la revendication 1, caractérisé en ce qu'on ajoute la trialkylamine aux autres partenaires réactionnels préalablement déposés.

3. Procédé suivant la revendication 1 ou 2, caractérisé en ce qu'on effectue la réaction avec un excès d'oxyde de chloralkyléthylène, calculé sur la trialkylamine utilisée.

4. Procédé suivant une des revendications 1 à 3, caractérisé en ce qu'on effectue la réaction avec un excès de tétrachloréthane, calculé sur la trialkylamine utilisée.

5. Procédé suivant une des revendications 1 à 4, caractérisé en ce qu'on effectue la réaction dans un solvant organique.